# EUROPEAN PATENT SPECIFICATION

(11) **EP 1 585 478 B1**
(45) Date of publication and mention of the grant of the patent: **17.03.2010**
(21) Application number: 03808560.1
(22) Date of filing: 23.12.2003
(51) Int. Cl.: A61K 9/00, A61K 38/43, A61L 2/00

(54) **USE OF SYNERGISTIC BACTERIOPHAGE LYTIC ENZYMES FOR PREVENTION AND TREATMENT OF BACTERIAL INFECTIONS**
VERWENDUNG VON SYNERGISTISCHEN LYTISCHEN ENZYMEN AUS BAKTERIOPHAGEN ZUR VORBEUGUNG UND BEHANDLUNG BAKTERIELLER INFEKTIONEN
UTILISATION D'ENZYMES LYTIQUES BACTERIOPHAGES SYNERGIQUES POUR LA PREVENTION ET LE TRAITEMENT D'INFECTIONS BACTERIENNES

(30) Priority: 23.12.2002 US 436077 P
(43) Date of publication of application: 19.10.2005
(73) Proprietor: THE ROCKEFELLER UNIVERSITY, New York, New York 10021-6399 (US)
(72) Inventor: FISCHETTI, Vincent, A., West Hempstead, NY 11552 (US); LOEFFLER, Jutta, New York, NY 10021 (US)
(74) Representative: Mercer, Christopher Paul
(86) International application number: PCT/US2003/041229
(87) International publication number: WO 2004/058182

(56) References cited:
- WO-A-01/50872
- US-A- 4 957 686
- US-A- 5 688 501
- US-B1- 6 254 866
- US-B1- 6 277 399
- LOEFFLER J M ET AL: "Rapid killing of Streptococcus pneumoniae with a bacteriophage cell wall hydrolase" SCIENCE, AMERICAN ASSOCIATION FOR THE ADVANCEMENT OF SCIENCE,, US, vol. 294, no. 5549, 7 December 2001 (2001-12-07), pages 2170-2172, XP002392510 ISSN: 0036-8075
- MONTERROSO BEGOÑA ET AL: "Crystallization and preliminary X-ray diffraction studies of the complete modular endolysin from Cp-1, a phage infecting Streptococcus pneumoniae." ACTA CRYSTALLOGRAPHICA. SECTION D, BIOLOGICAL CRYSTALLOGRAPHY SEP 2002, vol. 58, no. 9, September 2002 (2002-09), pages 1487-1489, XP002445858
- DATABASE MEDLINE US NATIONAL LIBRARY OF MEDICINE (NLM 1678400), BETHESDA, MD, US; THE JOURNAL OF INFECTIOUS DISEASES SEP 1991 COONROD J D ET AL: "Mechanism of killing of pneumococci by lysozyme." XP002445859
- SHEEHAN ET AL.: 'The lytic enzyme of the pneumococal phage Dp-1:a chimeric lysin of intergeneric origin.' MOLECULAR MICROBIOLOGY. vol. 25, no. 4, August 1997, pages 717 - 725, XP000922620
- NELSON ET AL.: 'Prevention and elimination of upper respiratry colonization of mice by group A streptococci by using a bacteriophage lytic enzyme.' PROC. NATL. ACAD. SCI. vol. 98, no. 7, 27 March 2001, pages 4107 - 4112, XP002165225
- SULAKVELIDZE ET AL.: 'Minireview: Bacteriohage therapy.' ANTMICROB. AGENTS CHEMOTHER. vol. 208, no. 3, March 2001, pages 649 - 659, XP001016193

## Description

### FIELD OF THE INVENTION

The invention relates to compositions comprising a combination of at least two bacteriophage lytic enzymes, wherein said enzymes ar Cpl-1 and Pal, and its used for the treatment of bacterial infections or for sanitizing or decontaminating porous or non-porous surfaces.

### BACKGROUND OF THE INVENTION

*Streptococcus pneumoniae* colonize the nasopharynx in many adults and even more children (50% and up), where they are believed to have their reservoir (Robinson et al. 2001, JAMA 285(13): 1729-1735). Elimination or reduction of those colonizing organisms could have a major impact on the occurrence of local and systemic pneumococcal disease. New conjugate vaccines for children are being tested extensively and reveal a number of unsolved problems, such as the choice of included serotypes and serotype replacement in colonized children (Pelton et aL 2000, Vaccine 19 Suppl. 1:S96-99). The use of a substance that can specifically eliminate all serotypes of *S. pneumoniae* from the nasopharynx is likely to reduce the bacterial load in the community and enhance herd immunity in combination with a vaccine (Barbour et aL 1995, J. Infect Dis. 171(1):93-98).

A substance has been identified that is able to accomplish this task. This substance, which is designated Pal, is an enzyme from a pneumococcal bacteriophage that can specifically digest any pneumococcal cell wall within seconds, resulting in rapid death of the organism (Loeffler et al. Science 294(5549):2170-2172).

Several such enzymes are known for pneumococcal bacteriophage, and are classified into two groups: Amidases, which cleave the peptidoglycan between N-acetylmuramic acid and L-alanine, and muramidases, such as lysozyme, which cleave the glycosidic bond between N-acetylmuramic acid and N-acetylglucosamine (Garcia et al. 1997, Microb. Drug Resist. 3(2):165-176). Cleavage with either of these enzymes results in a weakening in the cell wall, which leads to the externalization of the cytoplasmic, membrane and ultimate lysis.

### SUMMARY OF THE INVENTION

The present invention provides the use of a therapeutically effective synergistic combination of bacteriophage derived lytic enzymes Cpl-1 and Pal for use in the treatment and prevention of bacterial diseases and infections, including those diseases and infections that are resistant to treatment with antibiotics such as, but not limited to, penicillin.
The infections to be treated are any infections caused by a bacterium whose cell wall contains peptidoglycan. These include both gram positive and gram negative organisms. In a preferred embodiment, the infections are caused by a streptococcus. In a further preferred embodiment, the infections are caused by *Streptococcus pneumoniae:*

The at least two bacteriophage derived lytic enzymes used in the present invention, the amidase Pal and the muramidase (lysozyme) Cpl-1. The amidase Pal has a molecular weight (MW) of 34kD and the lysozyme Cpl-1 is a phage lysozyme with a MW of 39kD. When combined together, the two enzymes exhibit synergistic bacteriocidal or bacteriostatic activity *in vitro* and *in vivo*.

In a yet further preferred embodiment, the lytic enzymes decrease the occurrence or severity of local and systemic pneumococcal disease. In another preferred embodiment, the lytic enzymes prevent or eliminate pneumococcal colonization.

The synergistic lytic enzymes are isolated from the phage of disease-causing bacteria. The lytic enzymes are preferably isolated from gram positive bacteria. More preferably, the lytic enzymes are isolated from Streptococci.

*In vitro* use of the bacteriophage derived synergistic lytic enzymes enhance the cleavage of the target bacterial peptidoglycan, which will ultimately result in destruction of the bacterial cell wall and hence show synergistic bacteriocidal or bacteriostatic activity.

The use of a combination of lytic enzymes stated above would prove effective in a hospital setting, wherein areas suspected of harboring infectious disease-causing organisms might be prevalent.

A further embodiment, of the present invention includes use of a combination of lytic enzymes stated above in the method for decontamination of inanimate objects and surfaces, for example, surgical instruments.
The present invention extends to the use of the lytic enzymes of the invention for the preparation of a composition for the prevention and elimination of bacterial colonization.

The combination of at least two bacteriophage derived lytic enzymes, stated above in the pharmaceutical compositions along with pharmaceutically acceptable carriers for treatment and prevention of local and systemic bacterial infections, and to provide for efficient prevention and elimination of bacterial colonization. In a preferred embodiment, the local and systemic bacterial infections to be treated with the compositions of the present invention are any bacteria having peptidoglycan in their cell wall, including but not limited to, streptococcal organisms. In a further preferred embodiment, the streptococcal infections to be treated are caused by *Streptococcus pneumoniae*.

The pharmaceutical composition comprising the combination of lytic enzymes, Pal and Cpl-1, may take into account the nature of the infection, and will determine whether the best mode for delivery to the site of the infection is via intravenous delivery, intramuscular delivery, subcutaneous delivery, or other means for providing the most effective and expeditious treatment of the infection.

The invention further provides for an anti-microbial composition for sanitizing or decontaminating porous or non-porous surfaces comprising at least two bacteriophage derived synergistic lytic enzymes stated above.

A further embodiment of the invention provides for an antimicrobial composition suitable for decontaminating inanimate solid surfaces suspected of containing infectious bacteria by treating the surfaces with a bacteriocidal or bacteriostatically effective amount of the antimicrobial composition. Correspondingly, the present invention extends to the use of the lytic enzymes of the invention for the preparation of a composition for the decontamination of inanimate objects and surfaces,
such as surgical instruments or other equipment used in various treatment regimens, such as dialysis equipment, in a hospital setting, prior to use of these objects or instruments for treating patients, or porous surfaces e.g. textiles, carpeting.

Another preferred embodiment provides for a composition comprising the combination of the at least two synergistic bacteriophage lytic enzymes stated above useful for sanitizing or decontaminating porous surfaces e.g. textiles, carpeting. Furthermore, the composition of the at least two bacteriophage lytic enzymes stated above may be used to decontaminate veterinarian surgical or examination areas, where such areas may be thought to harbor infectious organisms susceptible to the bacteriostatic or bacteriocidal activity of the above stated combination.

In a further embodiment, the combination of at least two bacteriophage derived lytic enzymes may be combined with other bacteriostatic or bacteriocidal agents useful for decontamination of inanimate solid surfaces suspected of containing infectious bacteria, or for decontamination of porous surfaces.

The invention further provides for a screening method for identifying agents capable of enhancing the activity of Pal and Cpl-1, comprising:
(a) preparing purified Pal and Cpl-1;
(b) contacting the Pal and Cpl-1 to a bacteria having radioactively labeled peptidoglycan in the cell wall in the presence or absence of a test compound under conditions which allow binding to the peptidoglycan; and
(c) determining the amount of peptidoglycan cleavage,
wherein an agent capable of enhancing Pal and Cpl-1 activity is identified when the release of radioactivity is enhanced in the presence but not the absence of the agent.

Other advantages of the present invention will become apparent from the ensuing detailed description taken in conjunction with the following illustrative drawings.

### Brief Description of the Drawings

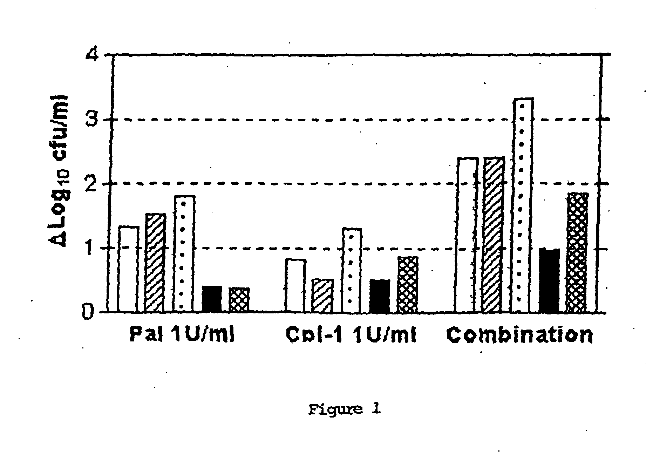

**Figure 1** shows the killing of 5 strains of *S. pneumoniae* with 1U/ml Pal, Cpl-1 or a combination of both in 30 seconds (DCC 1335, open bar; DCC 1355, hatched; DCC 1420, stippled; DCC 1494, filled, DCC 1490, cross-hatched). The combination of the two enzymes shows more than additive killing on a logarithmic scale.

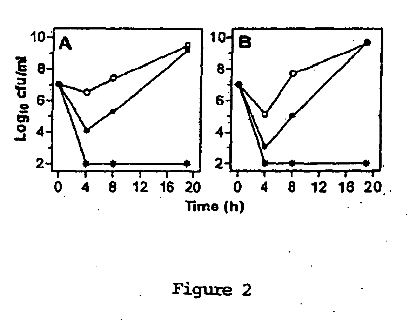

**Figure 2** shows time-kill curves for *S. pneumoniae* strains DCC 1355 (A) and DCC 1494 (B) with Pal (filled circles), Cpl-1 (open circles) and a combination of both (stars) at 0.25 [minimal inhibitory concentration (MIC)] each (12.5 U/ml Pal and Cpl-1 for DCC 1355, 12.5U /ml Pal and 6.25 U/ml Cpl-1 for DCC 1494).

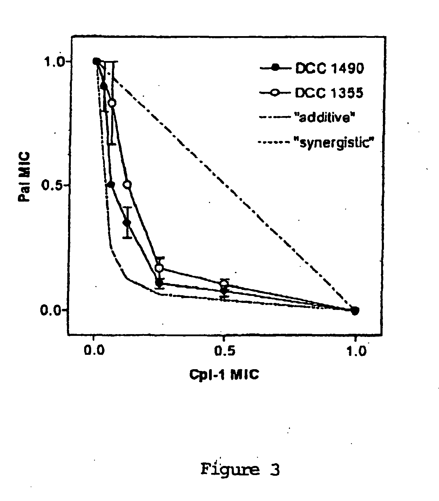

**Figure 3** shows an isobologram of the checkerboard synergy testing method, showing results for *S. pneumoniae* strains DCC 1490 and DCC 1355. For each well along the inhibitory line, enzyme concentrations (fractions of their MIC) were entered in an X/Y plot. Error bars show standard error of means. The two dashed lines illustrate theoretical curves.

### DETAILED DESCRIPTION OF THE INVENTION

Before the present methods and treatment methodology are described, it is to be understood that this invention is not limited to particular methods, and experimental conditions described, as such methods and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" include one or more methods, and/or steps of the type described herein and/or which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described. All publications mentioned herein are incorporated herein by reference in their entirety.

### Definitions

"Treatment" refers to the administration of medicine or the performance of medical procedures with respect to a patient, for either prophylaxis (prevention) or to cure the infirmity or malady in the instance where the patient is afflicted.

A "therapeutically effective amount" is an amount sufficient to decrease or prevent the symptoms associated with the bacterial infection.

A "prophylactically effective amount" is an amount sufficient to prevent a bacterial infection or the symptoms associated with the infection.

A "bacteriostatic" amount as used herein, is used to describe an amount of the combination of each of the synergistic enzymes, or a composition comprising the at least two, or the at least three, or the at least four, synergistic enzymes, sufficient to inhibit the growth of the infectious organisms.

A "bacteriocidal" amount as used herein, is used to describe an amount of the combination of each of the synergistic enzymes, or a composition comprising the at least two, or the at least three, or the at least four, synergistic enzymes, sufficient to kill the infectious organisms.

"Synergy" or "synergistic" amount or activity refers to the effects of a combination of antimicrobial agents (such as the lytic enzymes described herein), wherein the antimicrobial activity of the combination is greater than the sum of the activity of the individual antimicrobial agents.

"Peptidoglycan" is a thick rigid layer that is found in both gram positive and gram negative bacterial cells. It is composed of an overlapping lattice of two sugars that are crosslinked by amino acid bridges. The exact molecular makeup of these layers is species specific. The two sugars are N-acetyl glucosamine (NAG) and N-acetyl muramic acid (NAM). Attached to NAM is a side chain generally of four amino acids. The crossbridge is most commonly composed of L-alanine, D-alanine, D-glutamic acid, and diamino pimelic acid (DPA). The D-amino acids are different than the L-amino acids found in proteins. D-amino acids have the identical structure and composition as L-amino acids except that they are mirror images of the L amino acids. Most biological systems have evolved to commonly handle only the L form of compounds. Bacteria however use the D-aminoacids in their cell walls and have enzymes called racemases to convert between D and L forms. The NAM, NAG and amino acid side chain form a single peptidoglycan unit that can link with other units via covalent bonds to form a repeating polymer. The polymer is further strengthened by cross links between the D-glutamic acid of one unit and DPA of the next glycan tetrapeptide. In some gram positive microbes there is often a peptide composed of glycine, serine and threonine in between the crossbridges. The degree of cross-linking determines the degree of rigidity. In gram positive cells the peptidoglycan is a heavily cross-linked woven structure that wraps around the cell. It is very thick with peptidoglycan accounting for 50% of the weight of the cell and 90% of the weight of the cell wall. Electron micrographs show the peptidoglycan to be 20-80 nm thick. In gram negative bacteria the peptidoglycan is much thinner with only 15-20% of the cell wall being made up of peptidoglycan which is only intermittently cross-linked. In both cases the peptidoglycan can be thought of as a strong, woven mesh that holds the cell shape. It is not a barrier to solutes, the openings in the mesh are large and all types of molecules can pass through them. The cell wall is the site of action of many important antibiotics and antibacterial agents.

An "amidase" is an enzyme that catalyzes the hydrolysis of an amide to an acid and ammonium.

A "muramidase" is a glycosidase enzyme that hydrolyzes the bond between N-acetyl muramic acid and N-acetylglucosamine.

A "glucosaminidase" or "β-N-acetylglucosaminidase" is an enzyme that cleaves all non-reducing terminal β-linked N-acetylglucosamine.

"Lysozyme" is a muramidase that hydrolyzes the bond between N-acetyl muramic acid and N-acetyl glucosamine, thus cleaving an important polymer of the cell wall of many bacteria.

An "endopeptidase" refers to any of a large group of enzymes that catalyze the hydrolysis of peptide bonds in the interior of a polypeptide chain or protein molecule.

"Colonization" refers to the presence of bacteria within a persons body, however, the bacteria do not cause a disease.

### General Description

The present invention provides the use of the above stated combination for treating or preventing bacterial infections, due to administering to a subject in need of such therapy a therapeutically effective or a prophylactically effective amount of combination of bacteriophage derived lytic enzymes Cpl-1 and Pal and a suitable carrier for delivery of the lytic enzymes to the site of infection. While an additive anti-microbial effect of the enzymes may have been predicted, it was not until the time of the present invention that the unexpected synergistic antimicrobial effect using at least two different lytic enzymes obtained from a bacteriophage was demonstrated.

The pharmaceutical composition comprising the above stated composition is for treating patients suffering from bacterial infections, or for preventing bacterial infections in patients susceptible to infections with bacteria having peptidoglycan in their cell wall. The anti-microbial compositions for sanitizing or decontaminating inanimate objects and surfaces comprising a combination of any of the lytic enzymes described herein are also objects of the present invention.

Pal and Cpl-1, two purified bacteriophage lytic enzymes, were tested for their *in vitro* activity, alone and in combination, against several serotypes of *Streptococcus pneumoniae*, including penicillin-resistant strains. The enzymes demonstrated synergy in their ability to cleave the bacterial peptidoglycan and thus may be more efficient for the prevention and elimination of pneumococcal colonization.

The effect of the two bacteriophage lytic enzymes described herein provides a new mechanism for killing bacterial cells due to their combined synergistic lytic effect on the cell walls of both penicillin sensitive and penicillin resistant streptococci. This combination may play an important role in cleaving the peptidoglycan in bacterial cell walls and thus may be more efficient for the prevention and elimination of pneumococcal colonization. The findings in the present application suggest that a search for other combinations of this type of enzyme, or for mimics of the dual enzymatic and synergistic activity may prove to be a useful strategy for identifying a new class of antimicrobial agents and treatment modalities.

In a specific embodiment, the Cpl-1 expressing *E. coli* DH5α(pJML6) was constructed as follows: The Cpl-1 gene was amplified from Cpl-1 total phage DNA with a primer pair designed according to the published GenBank sequence number Z47794, flanked by XbaI and HindIII restriction sites, transcriptional start and stop codons, and a ribosomal binding site. Activity of the enzyme was also monitored by exposing *S. pneumoniae* strain DCC 1490 (serogroup 14), grown to log phase and resuspended in sterile saline, to an equal volume of serial 2-fold dilutions of the enzymes for 15 minutes in a microtiter plate. The reciprocal of the highest dilution that decreased the optical density by half (from a starting point of 0.5) was defined as the activity in U/ml. The specific activity of a freshly produced and purified batch for both enzymes is approximately 1U/ug.

### Therapeutic Uses of the Invention

The antimicrobial activity of the synergistic lytic enzymes of the present invention advantageously permits treatment of bacterial infection in any animal, particularly mammals, and more particularly humans. Other animals that can be treated include, but are not limited to, pets (dogs, cats, rodents, ferrets, etc.); laboratory animals (rats, mice, rabbits, hamsters, guinea pigs, etc.); farm animals; and wild animals, e.g., in a zoo.

The synergistic lytic enzymes of the present invention may be used therapeutically to inhibit the growth of bacteria comprising cell walls that are susceptible to the activity of the synergistic lytic enzymes. The methods of this invention may also be applied prophylactically to prevent a patient from becoming sick after an exposure to a potentially infectious bacterium. Without being bound to a theory, this growth inhibition may occur by cleavage of the chemical bonds within the peptidoglycan structure of the bacterial cell wall. This cleavage may weaken the cell wall structure to physical and osmotic stresses, thereby increasing chances for cell wall rupture. This invention may include embodiments wherein bacterial infections of the respiratory tract, nasal cavity, the throat, the mouth and the sinus cavities are treated. Other methods which accomplish delivery of the synergistic lytic enzyme-containing compositions to any site of infection are also suitable and are possible embodiments of the present invention.

The at least two lytic enzymes of the present invention can also be used successfully prophylactically on medical instruments, e.g., as a sterilization agent. When applied to catheters, stents, artificial joins, pins, and other implanted devices, the synergistic lytic enzymes prevent development of infections. In certain applications, the lytic enzymes may be implanted by including recombinant cells that produce the protein in the device, e.g., for coronary or peripheral arterial shunts.

In a preferred embodiment, the foregoing synergistic lytic enzymes provide for use in the prophylaxis and treatment of pneumonia, particularly in a hospital setting.

### Therapeutic and Prophylactic Compositions and Their Use

The invention provides composition for use in treatment comprising administering to a subject an effective amount of a combination of the invention. In a preferred aspect, the compound is substantially purified (*e*.*g*., substantially free from substances that limit its effect or produce undesired side-effects). The patient or subject is preferably an animal, including but not limited to animals such as monkeys, cows, pigs, horses, chickens, cats, dogs, etc., and is preferably a mammal, and most preferably human. In one specific embodiment, a non-human mammal is the subject. In another specific embodiment, a human mammal is the subject.

Various delivery systems are known and can be used to administer a compound of the invention, *e*.*g*., encapsulation in liposomes, microparticles, or microcapsules. Methods of introduction can be enteral or parenteral and include but are not limited to intradermal, intramuscular, intraperitoneal, intravenous, subcutaneous, intranasal, epidural, topical and oral routes. The compounds may be administered by any convenient route, for example by infusion or bolus injection, by absorption through epithelial or mucocutaneous linings (*e*.*g*., oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e*.*g*., by use of an inhaler or nebulizer, and formulation with an aerosolizing agent. In a specific embodiment, it may be desirable to administer the pharmaceutical compositions of the invention locally to the area in need of treatment, such as topical use on the skin; any suitable method known to the art may be used.

Another aspect of the invention provides for pharmaceutical compositions comprising purified Pal and Cpl-1 for therapeutic use in treatment and/or prevention of bacterial infections. One embodiment features treatment of a wide range of infections including those caused by gram positive, gram negative or mycobacterial infection with pharmaceutical compositions containing acceptable carriers and excipients. Moreover, a further embodiment may include a pharmaceutical composition designed for use in topical treatment of bacterial infections. Another embodiment may include a pharmaceutical composition designed for use in treatment of systemic infections, or infections that are non-responsive to other antibiotic modalities.

Such compositions comprise a therapeutically effective amount of an agent, and a pharmaceutically acceptable carrier. In a particular embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the subject. The formulation should suit the mode of administration.

The present invention thus provides a method of enhancing the antimicrobial effects of bacteriophage derived lytic enzymes, which, when used alone, show the benefit of killing certain bacteria through their deleterious effect on the peptidoglycan in the cell wall. When administering an amidase Pal, together with a lysozyme, Cpl-1, the effects of this combination are synergistic rather than additive, as shown in the present application. Thus, the compositions of the instant application provide for a combined preparation for simultaneous or sequential administration for the treatment of or the prevention of bacterial infections.

Preferably, this administration of the synergistic lytic enzymes is repeated over a period of several or multiple days.

The amidase Pal is used concurrently with the muramidase, (lysozyme) Cpl-1

The amount of amidase Pal to be used as described in the present invention varies according to the degree of the effective amount required for treating specific bacterial infections. The amount of lysozyme Cpl-1 to be used as described in the present invention also varies according to the degree of the effective amount required for treating specific bacterial infections. When combined together in the same composition, the preferred amount of each will be titrated to achieve the desired effective dose level. Procedures for titrating the most effective dose level of each enzyme are known to those skilled in the art.

The bacterial infections for which a composition containing Pal and Cpl-1 is a particularly suitable treament include infections caused by streptococcus, including *S. pneumoniae*, although the invention should not be construed to be limited to one particular organism.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection.

Also included are solid form preparations which are intended to be converted, shortly before use, to liquid form preparations for either oral or parenteral administration. Such liquid forms include solutions, suspensions and emulsions.

The lytic enzymes of the present invention may also be deliverable transdermally. The transdermal compositions can take the form of creams, lotions, aerosols and/or emulsions and can be included in a transdermal patch of the matrix or reservoir type as are conventional in the art for this purpose.

Preferably, the pharmaceutical preparation is in unit dosage form. In such form, the preparation is subdivided into unit doses containing appropriate quantities of the active component, e.g., an effective amount to achieve the desired purpose.

The actual dosage employed may be varied depending upon the requirements of the patient and the severity of the condition being treated. Determination of the proper dosage for a particular situation is within the skill of the art. For convenience, the total daily dosage may be divided and administered in portions during the day, if desired.

The combination of lytic enzymes may be administered using conventional techniques such as oral administration. Intravenous administration is preferable for a continuous dosing therapy regimen. Oral administration can be utilized for a repeat dosing regimen.

The amidase Pal can be administered separately prior to, or concurrent with the lysozyme Cpl-1. The amidase part can be administered with Cpl-1 as unit dosage form to facilitate patient dosing. Such combination dosage forms may be in any of the above-described dosage forms, but, as noted above, are preferably in oral or intravenous forms.

The amidase and the lysozyme can be packaged in a kit form. In such a kit, the amidase and the lysozyme would be individually formulated into particular dosage forms for the particular route of administration, and contain instructions for the administration of the contents. In a typical embodiment for oral formulation, such a kit may be in the form of a blister package with separately formulated oral dosage forms of the amidase and the lysozyme.

Any necessary adjustments in dose can be readily made to meet the treatment requirements of the individual patient and adjusted accordingly by the skilled practitioner.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The amount of the compound of the invention which will be effective in the treatment of infectious diseases wherein the bacteria contain the Pal and Cpl-1 enzymes can be determined by standard clinical techniques based on the present description. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. Effective doses may be extrapolated from dose-response curves derived from *in vitro* or animal model test systems.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

In a specific embodiment, it may be desirable to administer the pharmaceutical compositions of the invention locally to the area in need of treatment; this may be achieved, for example, and not by way of limitation, by local infusion during surgery, by topical application, by injection, by means of a catheter, or by means of an implant, said implant being of a porous, non-porous, or gelatinous material, including membranes, such as sialastic membranes, or fibers or co-polymers such as Elvax (see Ruan et al , 1992, Proc Natl Acad Sci USA, 89:10872-10876). In one embodiment, administration can be by direct injection by aerosol inhaler.

In another embodiment, the Pal and Cpl-1 combination can be delivered in a vesicle, in particular a liposome (see Langer (1990) Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.)

In yet another embodiment, the Pal and Cpl-1 combination can be delivered in a controlled release system. In one embodiment, a pump may be used (see Langer, *supra*; Sefton (1987) CRC Crit. Ref. Biomed. Eng. 14:201; Buchwald et al. (1980) Surgery 88:507; Saudek et al. (1989) N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J. (1983) Macromol. Sci. Rev. Macromol. Chem. 23:61; see also Levy et al. (1985) Science 228:190; During et al. (1989) Ann. Neurol. 25:351; Howard et al. (1989) J. Neurosurg. 71:105). In yet another embodiment, a controlled release system can be placed in proximity of the therapeutic target, i.e., the airways, thus requiring only a fraction of the systemic dose (see, *e*.*g*., Goodson, in Medical Applications of Controlled Release (1984) supra, vol. 2, pp. 115-138). Other suitable controlled release systems are discussed in the review by Langer (1990) Science 249:1527-1533.

### Effective Dose

The lytic enzymes described herein can be administered to a patient at therapeutically effective doses to treat certain diseases or disorders. A therapeutically effective dose refers to that amount of a therapeutic sufficient to result in a healthful benefit in the treated subject. A prophylactically effective dose refers to that amount of a prophylactic sufficient to result in prevention of a disease in a patient.

The precise dose of the therapeutic embodied by this invention, to be employed in the formulation, will depend on the route of administration, and the nature of the patient's disease, and should be decided according to the judgment of the practitioner and each patient's circumstances according to standard clinical techniques. The term "inhibit" or "inhibition" means to reduce by a measurable amount. Experimental evidence of inhibition may include observing the elimination of a bacterial infection in an animal model. Effective doses may thus be extrapolated from dose-response curves derived from animal model test systems.

Toxicity and therapeutic efficacy of the lytic enzymes can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀ /ED₅₀. Therapeutics that exhibit large therapeutic indices are preferred. While therapeutics that exhibit toxic side effects can be used, care should be taken to design a delivery system that targets such compounds to the site of affected tissue in order to minimize potential damage to uninfected cells and, thereby, reduce side effects.

The data obtained from the *in vitro* bacterial growth inhibition and cell lysis assays and animal studies can be used in formulating a range of dosage for use in humans. The dosage of lytic enzymes containing compositions lies preferably within a range of concentrations that include the ED₅₀ with little or no toxicity. The dosage can vary within this range depending upon the dosage form employed and the route of administration utilized. For any composition used in the method of the invention, the therapeutically effective dose can be estimated initially from *in vitro* bacterial growth inhibition and cell lysis assays. A dose can be formulated in animal models to achieve a lytic enzyme concentration range that includes the IC₅₀ (i.e., the concentration of the test compound which achieves a half-maximal inhibition of symptoms). Such information can be used to more accurately determine useful doses in humans. The efficacy of a particular dosage in eliminating, for example, a bacterial throat infection may be measured by a traditional throat culture swab assay. Any method which provides data as to the number of bacteria present at the site of infection would be suitable for these purposes.

In order to be effective, the enzymes should be present in an amount sufficient to provide an effective number of enzyme units in contact with the oral cavity or respiratory tract. Having too few enzyme units in contact with the oral cavity or the respiratory tract, even over a long period of time, will not produce as beneficial an effect as desired. Hence, any dosage form employed should provide for an approximate minimum number of units for the amount of time that the dosage will provide enzyme to the oral cavity or the respiratory tract. The concentration of the active units of enzymes believed to provide for an effective amount or dosage of enzymes may be in the range of from about 100 units to about 100,000 units in the environment of the nasal and oral passages. Within that broader range, dosages of from about 100 units to about 10,000 units are believed to be acceptable. Such units can be contained in smaller volumes of carrier such as liquid or saliva, e.g. 1 ml or less (e.g. in the case of a lozenge) or can be contained in larger dosage volumes such as a gargle of several mls. Generally, therefore, larger volumes of carrier will require a greater total number of units to achieve an effective concentration of active enzyme. Hence, acceptable concentrations can be from about 100 units/ml to about 100,000 units/ml of fluid in the environment of the nasal or oral passages. Within this range, concentrations from about 100 units/ml to about 10,000 units/ml are acceptable.

In practice, therefore, the time exposure to the active enzyme units likely will influence the desired concentration of active enzyme units employed in the dosage per ml. For example, carriers that are considered to provide prolonged release (certain nasal sprays, lozenges and encapsulated enzyme) could provide a lower concentration of active enzyme units per ml, but over a longer period of time. Conversely, a shorter duration treatment (e.g., a gargle) could provide a higher concentration of active enzyme units per ml. Any dosage form containing sufficient lytic enzyme to provide effective concentrations of active enzyme at the site of infection or to provide a sufficient prophylactic effect are well within the bounds of routine experimentation and therefore, well within the scope of the instant invention.

### Compositions for Sanitizing or Decontaminating Inanimate Porous or Non-porous Objects

In yet another embodiment, the composition of the present invention is envisioned for sanitizing or decontaminating inanimate non-porous or porous objects using a bactericidal or bacteriostatically effective amount of the combination of the at least two synergistic bacteriophage lytic enzymes, wherein said at least two enzymes are Cpl-1 and Pal. The composition of the present invention for this particular use may be employed for reducing the microbial population of surgical instruments, surgical gowns, examination areas, and other medical or veterinarian tools or instruments.

It is also envisioned that the composition of the present invention may be employed for disinfecting dental instruments. Thus, the composition containing Pal and Cpl-1 can prevent a potential infection which may arise from the use of instruments or equipment that are contaminated with bacteria which may be susceptible to the killing activity of Pal and Cpl-1. Furthermore, the amount of each lytic enzyme for use in the composition would be determined through standard methods of titration for anti-microbial activity known to those skilled in the art.

Although the antibacterial property of lysozymes in general has been well documented (Procter et al in CRC Crit. Reviews in Food Science and Nutrition, 1988, 26(4):359-395), the synergistic anti-microbial effect of lysozyme, Cpl-1, in combination with bacteriophage amidase Pal, has not been disclosed. It is with regard to this unexpected finding that the present invention is directed.

The compositions of the present invention for sanitizing or for disinfectant use may include other additives. Examples of such additives include one or more anti-microbial agents, including other lytic enzymes, or bacteriocins, or chelating agents. Other agents that enhance the permeability of the antibacterial agents or lytic enzymes are also contemplated.

Furthermore, the composition may include other agents that function to stabilize the bacteriophage derived lytic enzymes. It is envisioned that the composition may be provided in liquid form, as a spray, as a gel, as a foam, or as a powder.

The compositions of the present invention may include surfactants such as an alkyl glycoside or an alkenyl glycoside, an alkoxylated alcohol, an alkoxylated carboxylic acid, a sorbitan ester, a polyethoxylated derivative of a sorbitan ester, a polyglycerol ester, a sucrate, an ester of fatty acid with a polyalcohol and/or a polyalkylene glycol. Other additives such as thickeners, preservatives, foaming agents may also be present in the composition.

In order to accelerate treatment of the infection, the administration of a second therapeutic agent may be an embodiment of this invention. The second therapeutic agent may be administered to the patient as a part of the lytic enzyme composition or in the form of a separate composition which comprises only said second therapeutic agent. A non-limiting exemplary list of suitable second therapeutic agents includes penicillin, synthetic penicillins bacitracin, methicillin, cephalosporin, polymyxin, cefaclor. Cefadroxil, cefamandole nafate, cefazolin, cefixime, cefinetazole, cefonioid, cefoperazone, ceforanide, cefotanme, cefotaxime, cefotetan, cefoxitin, cefpodoxime proxetil, ceftazidime, ceftizoxime, ceftriaxone, cefriaxone moxalactam, cefuroxime, cephalexin, cephalosporin C, cephalosporin C sodium salt, cephalothin, cephalothin sodium salt, cephapirin, cephradine, cefuroximeaxetil, dihydratecephalothin, moxalactam, loracarbef mafate, chelating agents and any combinations thereof in amounts which are effective to further enhance the therapeutic effect of the lytic enzyme. Furthermore, antifingal compositions may be coadministered with the lytic enzyme containing compositions of this invention (separately or within the same composition as the lytic enzyme) Such antifungal agents may include Amphotericin B, Carbol-Fuchsin, Ciclopirox, Clotrimzole, Econazole, Haloprogin, Ketoconazole, Mafenide, Miconazole, Naftifine, Nystatin, Oxiconazole Silver, Sulfadiazine, Sulconazole, Terbinafine, Tioconazole, Tolnaftate, Undecylenic acid, flucytosine, miconazole, or others.

Compositions of the present invention may further comprise antiviral agents. Such antiviral agents may include zinc containing substances, such as zinc gluconate. Furthermore anesthetic agents may be included in the compositions of the present invention. Suitable anaesthetics may include aspirin, acetaminophen, phenol, benzocaine, diphenhydramine, kaolin-pectin and Xylocaine. Furthermore, decongestants and antihistamines may be included in compositions of the invention. Suitable decongestants may include pseudoephedrine, phenylpropanolamine and phenylephrine; antihistamines may include brompheniramine or chlorpheniramine.

### Screening Assays

The invention further provides a screening method. Such screening method is also envisioned for identifying agents capable of enhancing the activity of Pal and Cpl-1, comprising :
(a) preparing purified Pal and Cpl-1;
(b) contacting the Pal and Cpl-1 to a bacteria having radioactively labeled peptidoglycan in the cell wall in the presence or absence of a test compound under conditions which allow binding to the peptidoglycan; and
(c) determining the amount of peptidoglycan cleavage,
wherein an agent capable of enhancing Pal and Cpl-1 activity is identified when the release of radioactivity is enhanced in the presence but not the absence of the agent.

### EXAMPLES

The following examples are put forth so as to provide those of ordinary skill in the art with a complete disclosure and description of how to isolate and use the novel class of enzymes described herein, and to provide a suitable means for identifying and assaying the synergistic effects of this class of enzymes and development of pharmaceutical compositions for therapeutic use, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### EXAMPLE 1 Bacterial strain and culture

*E. coli* DH5α(pMSP11) expressing Pal and double-stranded DNA from the pneumococcal phage Cpl-1 were used in the present studies (Sheehan et al. 1997, Mol. Microbiol. 25(4): 717-725). The Cpl-1 expressing *E. coli* DH5α(pJML6) was constructed as follows: The Cpl-1 gene was amplified from Cpl-1 total phage DNA with a primer pair designed according to the published GenBank sequence number Z47794, flanked by XbaI and HindIII restriction sites, transcriptional start and stop codons, and a ribosomal binding site. To use the same powerful expression system as for Pal, which is based on the construct pIN-IIIA and contains a double promoter, we digested the plasmid pMSP11 with XbaI and HindIII, which removes the entire inserted Pal gene (Masui et al, 1984, Bio/Technology:81-85, Sheehan et al. 1997, Mol. Microbiol. 25(4): 717-725). The PCR product was subcloned to pMSP11 using the XbaI and HindIII recognition sites to produce the pJML6 construct. *E. coli* DH5α(pMSP11) and DH5α(pJML6) were grown in LB broth and induced with lactose overnight. Harvest and purification of the enzymes are described elsewhere (Loeffler et al, 2001, Science 294(5549):2170-2172).

### Enzyme Assay

Both enzymes were stored and assayed in 50mM phosphate buffer (pH 7.0) containing 1mM DTT and 1mM BDTA (hereafter called enzyme buffer). Activity was measured by exposing *S. pneumoniae* strain DCC 1490 (serogroup 14), grown to log-phase and resuspended in sterile saline, to and equal volume of serial 2-fold dilutions of the enzymes for 15 minutes in a microtiter plate. The reciprocal of the highest dilution that decreased the optical density by half (from a starting point of 0.5) was defined as the activity in U/ml. The specific activity of a freshly produced and purified batch for both enzymes is approximately 1U/µg.

### EXAMPLE 2 Time Kill Experiments-Short Exposure

Time-kill experiments were conducted with very short exposures, since the killing with both enzymes can be observed within seconds. Moreover, it is envisioned that a typical application of compositions containing a combination of both enzymes in the nasopharynx would be unique and short. Mid-log-phase cultures of *S. pneumoniae* strains DCC 1355, DCC 1490, DCC 1494, DCC 1335, and DCC 1420, (serotypes 19, 14, 14, 9V, 23F and, the latter 3 highly penicillin-resistant) were pelleted and resuspended to an absorbance at 600nm of 1.0 (approximately 10⁹ CFU/ml). 150 µl of Pal or Cpl-1 at a final concentration of 1 U/ml, or a mixture of both at a concentration of 0.5 U/ml each was added to 150 µl of the bacterial solution. Colony counts were performed after 30 seconds and 10 minutes and compared to a control exposed to enzyme buffer only, by serially diluting a 10 µl aliquot in saline and plating on 5% Columbia blood agar (CBA), with a detection limit of 10⁴ CFU/ml.

In 30 seconds 1U/ml Pal reduced the bacterial titer of the 4 strains by Log₁₀ CFU/ml (median range) 1.34 (0.38 - 1.81), while Cpl-1 at 1U/ml reduced the titers by log₁₀ CFU/ml 0.83 (0.52 - 1.31). The combination of both enzymes reduced the titers by log₁₀ CFU/ml 2.40 (0.98 - 3.34) (Figure 1). After 10 minutes, reduction was log₁₀ CFU/ml (median range) 1.99 (0.73 - 2.54), 1.44 (1.32 - 2.65), and 3.15 (2.50 - 5.28), for Pal, Cpl-1 and the combination respectively. In other words, mixing 0.5U/ml of each enzyme increased killing efficacy by log₁₀ 1.07 to 131 (median, 30s and 10 min), compared to Pal alone and by log₁₀ 1.58 to 1.72 (30s and 10 min), compared to Cpl-1 alone. Using one-way ANOVA and the Bonferroni post-test for the comparison of the three treatment groups, the killing efficacy of the combination of enzymes was always significantly higher than that of both single enzymes (p < 0.05), at both time points, except for strain DCC 1494 at 30 seconds.

### EXAMPLE 3 Time Kill Experiments-Long Exposure

Time-kill studies with longer exposure (up to 19h) were performed using only strains DCC 1355 and DCC 1494. Bacteria were grown in cation-adjusted Muller-Hinton broth (CAMHB) with 2.5% lysed horse blood to mid-log-phase and resuspended in fresh medium at a titer of 1x 10⁷ CFU/ml. Pal, Cpl-1 or a combination of both were added at 0.25 MIC (12.5U/ml of Pal and Cpl-1 for strain DCC 1355, 12.5 U/ml of Pal and 6.25 U/ml of Cpl-1 for strain DCC 1494). Samples were taken at 0, 4, 8 and 19 hours, serially diluted and plated on CBA for titer determination, with a detection limit of 100 CFU/ml. The usual definition of a titer reduction of the combination ≥ 2 log₁₀ greater than that of the single most active agent was used for determination of synergy. Figure 2 illustrates the results, which show synergy for both strains.

### EXAMPLE 4 Determination of Minimal Inhibitory Concentration

The well-described checkerboard broth microdilution method, which allows for the concurrent determination of the minimal inhibitory concentration (MIC) of each agent tested (Eliopoulos et al. 1991, Antimicrobial Combinations, V. Lorian Ed., Williams & Wilkins, Baltimore, MD), was then applied. Testing was performed repeatedly with all 5 strains described above in CAMHB supplemented with 5% lysed horse blood, with a final inoculum of 5 x 10⁴ CFU per well. Both enzymes were assayed at concentrations between 200 and 3.125 U/ml. Plates were incubated at 37°C for 18 h in ambient air and examined visually for growth using a reflective viewer. The fractional inhibitory concentration index (ΣPIC) [i.e., the sum of the fractions of both MICs that showed inhibition) was calculated. A ΣFIC of ≤ 0.5 was interpreted as synergy. The MICs were 50-200 U/ml for Pal and 25-50 U/ml for Cpl-1 in all tested strains. Transcription of the enzyme concentrations along the inhibitory line on the microtiter plate into an isobologram revealed curves with a characteristic shape of synergy. The results of strains DCC 1490 and DCC 1355 are shown in figure 3. The ΣFIC for all strains was ≤ 0.5.

The combination of the two lytic bacteriophage enzymes Pal and Cpl-1 appears to have synergistic activity on several *S. pneumoniae* strains, including those that are penicillin-resistant, *in vitro*. This positive interaction could either to be due to the increased access of these enzymes to the respective cleavage sites or to the enhanced destructive effect of a two-dimensional digestion in the three-dimensional peptidoglycan.

## Claims

1. A combination of:
(a) at least two bacteriophage-derived lytic enzymes; and
(b) a suitable carrier for delivery of the lytic enzymes to a site of bacterial infection for use in therapy,
wherein said at least two bacteriophage-derived lytic enzymes are Cpl-1 and Pal.

2. The use of at least two bacteriophage-derived lytic enzymes in the manufacture of a medicament for the prevention or treatment of bacterial infections, wherein said at least two bacteriophage-derived lytic enzymes are Cpl-1 and Pal.

3. A pharmaceutical composition comprising:
(a) at least two therapeutically-effective synergistic bacteriophage-derived lytic enzymes; and
(b) a carrier suitable for delivery of the lytic enzymes to the site of infection,
wherein said at least two bacteriophage-derived lytic enzymes arc Cpl-1 and Pal.

4. An anti-microbial composition for sanitizing or decontaminating porous or non-porous surfaces comprising at least two bacteriophage-derived synergistic lytic enzymes, wherein said at least two bacteriophage-derived lytic enzymes are Cpl-1 and Pal.

5. The combination, the use, the pharmaceutical composition or the anti-microbial composition of any preceding claim, wherein the bacterial infections are caused by *Streptococcus pneumoniae*.

6. The combination, the use, the pharmaceutical composition or the anti-microbial composition of any preceding claim, wherein the lytic enzymes decrease the occurrence or severity of local and systemic pneumococcal disease.

7. The combination, the use, the pharmaceutical composition or the anti-microbial composition of any preceding claim, wherein the lytic enzymes prevent or eliminate pneumococcal colonization.

8. The combination, the use, the pharmaceutical composition or the anti-microbial composition of any preceding claim, wherein said at least two lytic enzymes are isolated from the phage of disease-causing bacteria, and wherein said disease causing bacteria are gram positive bacteria.

9. The combination, the use, the pharmaceutical composition or the antimicrobial composition of claim 8, wherein the gram positive bacteria is *Streptococcus*.

10. The combination, the use, the pharmaceutical composition or the antimicrobial composition of claim 9, wherein the *Streptococcus* is *Streptococcus pneumoniae*.

11. A screening method for identifying agents capable of enhancing the synergistic activity of Pal and Cpl-1 combination, comprising:
(a) preparing purified Pal and Cpl-1;
(b) contacting the Pal and Cpl-1 to a bacteria having radioactively labelled peptidoglycan in the cell wall in the presence or absence of a test compound under conditions which allow binding to the peptidoglycan; and
(c) determining the amount of peptidoglycan cleavage,
wherein an agent capable of enhancing Pal and Cpl-1 combined activity is identified when the release of radioactivity is enhanced in the presence but not the absence of the agent.

12. A method for decontaminating inanimate surfaces suspected of containing infectious bacteria comprising treatment of said surfaces with a bacteriocidal or bacteriostatically effective amount of the anti-microbial composition of claims 4.

## Patentansprüche

1. Kombination von:
(a) mindestens zwei von Bakteriophagen stammenden lytischen Enzymen und
(b) einem geeigneten Träger für die Verabreichung der lytischen Enzyme an eine Stelle bakterieller Infektion,
zur Verwendung in der Therapie,
wobei die mindestens zwei von Bakteriophagen stammenden lytischen Enzyme Cpl-1 und Pal sind.

2. Verwendung von mindestens zwei von Bakterien stammenden lytischen Enzymen in der Herstellung eines Medikaments für die Vorbeugung oder Behandlung von bakteriellen Infektionen, wobei die mindestens zwei von Bakteriophagen stammenden lytischen Enzyme Cpl-1 und Pal sind.

3. Pharmazeutische Zusammensetzung, umfassend:
(a) mindestens zwei therapeutisch wirksame, synergistische von Bakteriophagen stammende lytische Enzyme und
(b) einen Träger, der für die Verabreichung der lytischen Enzyme an die Stelle der Infektion geeignet ist,
wobei die mindestens zwei von Bakteriophagen stammenden lytischen Enzyme Cpl-1 und Pal sind.

4. Antimikrobielle Zusammensetzung für das Desinfizieren oder Dekontaminieren poröser oder nicht-poröser Oberflächen, umfassend mindestens zwei von Bakteriophagen stammende synergistische, lytische Enzyme, wobei die mindestens zwei von Bakteriophagen stammenden lytischen Enzyme Cpl-1 und Pal sind.

5. Kombination, Verwendung, pharmazeutische Zusammensetzung oder antimikrobielle Zusammensetzung nach einem der vorhergehenden Ansprüche, wobei die bakteriellen Infektionen durch *Streptococcus pneumoniae* verursacht sind.

6. Kombination, Verwendung, pharmazeutische Zusammensetzung oder antimikrobielle Zusammensetzung nach einem der vorhergehenden Ansprüche, wobei die lytischen Enzyme das Auftreten oder die Schwere von lokaler und systemischer, durch Pneumokokken verursachter Erkrankung mindern.

7. Kombination, Verwendung, pharmazeutische Zusammensetzung oder antimikrobielle Zusammensetzung nach einem der vorhergehenden Ansprüche, wobei die lytischen Enzyme die Besiedlung mit Pneumokokken verhindern oder beseitigen.

8. Kombination, Verwendung, pharmazeutische Zusammensetzung oder antimikrobielle Zusammensetzung nach einem der vorhergehenden Ansprüche, wobei die mindestens zwei lytischen Enzyme aus dem Phagen von Krankheit verursachenden Bakterien isoliert sind und wobei die Krankheit verursachenden Bakterien gram-positive Bakterien sind.

9. Kombination, Verwendung, pharmazeutische Zusammensetzung oder antimikrobielle Zusammensetzung nach Anspruch 8, wobei die gram-positiven Bakterien *Streptococcus* sind.

10. Kombination, Verwendung, pharmazeutische Zusammensetzung oder antimikrobielle Zusammensetzung nach Anspruch 9, wobei der *Streptococcus Streptococcus pneumoniae* ist.

11. Screeningverfahren für das Identifizieren von Mitteln, die befähigt sind, die synergistische Aktivität der Kombination von Pal und Cpl-1 zu verbessern, umfassend:
(a) Herstellen von gereinigtem Pal und Cpl-1,
(b) Inkontaktbringen des Pal und Cpl-1 mit einem Bakterium, das radioaktiv markiertes Peptidoglykan in der Zellwand aufweist, in der Anwesenheit oder Abwesenheit einer Testverbindung unter Bedingungen, die Bindung an das Peptidoglykan ermöglichen, und
(c) Bestimmen der Menge der Peptidoglykanspaltung,
wobei ein Mittel, das befähigt ist, die kombinierte Aktivität von Pal und Cpl-1 zu verbessern, identifiziert wird, wenn die Freisetzung von Radioaktivität in der Anwesenheit, aber nicht in der Abwesenheit des Mittels, verbessert wird.

12. Verfahren zum Dekontaminieren von unbelebten Oberflächen, die im Verdacht stehen, infektiöse Bakterien zu enthalten, umfassend das Behandeln der Oberflächen mit einer bakteriozidal oder bakteriostatisch wirksamen Menge der antimikrobiellen Zusammensetzung nach Anspruch 4.

## Revendications

1. Combinaison :
(a) d'au moins deux enzymes lytiques dérivées d'un bactériophage ; et
(b) d'un véhicule approprié pour délivrer les enzymes lytiques à un site d'infection bactérienne pour une utilisation en thérapie,
dans laquelle lesdites au moins deux enzymes lytiques dérivées d'un bactériophage sont Cpl-1 et Pal.

2. Utilisation d'au moins deux enzymes lytiques dérivées d'un bactériophage dans la fabrication d'un médicament destiné à la prévention ou au traitement d'infections bactériennes, dans laquelle lesdites au moins deux enzymes lytiques dérivées d'un bactériophage sont Cpl-1 et Pal.

3. Composition pharmaceutique comprenant :
(a) au moins deux enzymes lytiques synergiques thérapeutiquement efficaces dérivées d'un bactériophage ; et
(b) un véhicule approprié pour délivrer les enzymes lytiques au site d'infection,
dans laquelle lesdites au moins deux enzymes lytiques dérivées d'un bactériophage sont Cpl-1 et Pal.

4. Composition anti-microbienne d'assainissement ou de décontamination de surfaces poreuses ou non poreuses comprenant au moins deux enzymes lytiques synergiques dérivées d'un bactériophage,
dans laquelle lesdites au moins deux enzymes lytiques dérivées d'un bactériophage sont Cpl-1 et Pal.

5. Combinaison, utilisation, composition pharmaceutique ou composition anti-microbienne selon l'une quelconque des revendications précédentes, dans laquelle les infections bactériennes sont provoquées par *Streptococcus pneumoniae*.

6. Combinaison, utilisation, composition pharmaceutique ou composition anti-microbienne selon l'une quelconque des revendications précédentes, dans laquelle les enzymes lytiques diminuent la survenue ou la gravité d'une maladie pneumococcique locale et systémique.

7. Combinaison, utilisation, composition pharmaceutique ou composition anti-microbienne selon l'une quelconque des revendications précédentes, dans laquelle les enzymes lytiques préviennent ou éliminent une colonisation pneumococcique.

8. Combinaison, utilisation, composition pharmaceutique ou composition anti-microbienne selon l'une quelconque des revendications précédentes, dans laquelle lesdites au moins deux enzymes lytiques sont isolées à partir du phage des bactéries provoquant la maladie, et dans laquelle lesdites bactéries provoquant la maladie sont des bactéries à Gram positif.

9. Combinaison, utilisation, composition pharmaceutique ou composition anti-microbienne selon la revendication 8, dans laquelle la bactérie à Gram positif est un *Streptococcus.*

10. Combinaison, utilisation, composition pharmaceutique ou composition anti-microbienne selon la revendication 9, dans laquelle le *Streptococcus* est *Streptococcus pneumoniae*.

11. Procédé de criblage pour identifier des agents capables de renforcer l'activité synergique de la combinaison de Pal et Cpl-1, comprenant :
(a) la préparation d'enzymes Pal et Cpl-1 purifiées ;
(b) la mise en contact de Pal et Cpl-1 avec une bactérie ayant un peptidoglycane marqué radioactivement dans la paroi cellulaire, en présence ou en l'absence d'un composé à tester, dans des conditions permettant une liaison au peptidoglycane ; et
(c) la détermination de la quantité de clivage du peptidoglycane,
dans lequel un agent capable de renforcer l'activité combinée de Pal et Cpl-1 est identifié lorsque la libération de radioactivité est améliorée en présence mais pas en l'absence de l'agent.

12. Procédé de décontamination de surfaces inanimées soupçonnées de contenir des bactéries infectieuses, comprenant le traitement desdites surfaces avec une quantité bactéricide ou efficace au niveau bactériostatique de la composition anti-microbienne selon la revendication 4.
